# EUROPEAN PATENT APPLICATION

(11) **EP 0 708 410 A1**
(43) Date of publication of application: **24.04.1996**
(21) Application number: 95112957.6
(22) Date of filing: 17.08.1995
(51) Int. Cl.: G06F 19/00

(54) **Method and device for analyzing large amounts of information**

(30) Priority: 21.10.1994 SE 9403608
(71) Applicant: Siemens Elema AB, S-171 95 Solna 1 (SE)
(72) Inventor: Lekholm, Anders, S-161 39 Bromma (SE)

(57) **Abstract**

In a method for analyzing large amounts of information, e.g. analysis of pictures, information carrying signals are processed, and corresponding information data are compressed in such a way that the original signals can be recreated by decompression of the data. The analysis of the information is performed on compressed data. A device for analyzing large amounts of data, e.g. analysis of pictures, comprises a unit (6, 8, 10, 12, 14) for acquiring, compressing and storing the information and a unit (30) for computer analysis of the compressed information.

## Description

The present invention relates to a method for analyzing large amounts of information, e.g. analysis of pictures, in which method information carrying signals are processed and corresponding information data are compressed in such a way that the original signals can be recreated by data decompression, and to a device for such an analysis, comprising a unit for the acquisition, compression and storage of the information.

Analysis, processing and storage of large amounts of information, such as picture information, are time-consuming and costly procedures.

A Holter ECG, i.e. a single or multi-channel ECG recorded over 24 hours, requires 20 Mb of storage space per channel.

The analysis of such an ECG was originally entirely manual, the physician then studying and analyzing each individual QRS, typically 100,000 QRS's per channel in a 24-hour recording.

Today, an automatic preanalysis is performed with the aid of a computer in which preanalysis different QRS morphologies are classified, and all QRS's are arranged in a number of classes which can range from one or a few dozen up to 256 or more. The task of the physician is then to analyze these classes. This computerized preanalysis obviously represents a considerable improvement in the efficiency of the physician's work.

The above described method was developed from the purely manual methods of the past. The analysis strongly resembles the analysis of individual QRS complexes made during e.g. an exercise ECG when the patient is briefly subjected to a defined workload while the heart activity of the patient is studied. The same or similar computer algorithms can be used. As the computer takes over the criteria and performs essentially the same evaluation which were previously performed manually the evaluation can be easily checked.

It has appeared that in the case of Holter ECG's about nine tenths of the original information is redundant and unimportant to interpretation of the ECG. By an appropriate data compression the amount of information could therefore be reduced to one tenth or less, which greatly reduces the cost of the recording equipment as semiconductor memories or other appropriate memories are relatively expensive. The compression of information would also greatly reduce the time required to download recorded ECG's from the portable recording unit to an analysis station.

Compressing and decompressing digitalized analog data take time and computer capacity. In the case of an ECG, the compression operation is no problem, since there is plenty of time to digitize and compress the relatively slowly varying analog ECG signal. However, information decompression is often an inconveniently slow process in present-day Holter ECG analysis stations. A typical Holter recording comprises two or three 24-hour channels, which means that the total volume of information amounts to 40 to 60 Mb, an amount of information so large that it takes a considerable time to process, even with a fast PC.

The time required to decompress data volumes of this magnitude ranges from 5 to 20 minutes, which approaches the time used by a physician for the analysis and diagnosis of the decompressed data volume. Before the physician's analysis, the computer also performs the aforementioned preanalysis which takes a few more minutes. The time required for decompression and preanalysis of the ECG information therefore becomes inconveniently long. A shortening of this time would increase the productivity considerably.

Another advantage of information compression is that much less time is needed to download information from the portable recording equipment to replay and analysis equipment.

A large number of methods for compressing and decompressing information are previously known.

Thus, EP,A2,0 552 806 describes a system with a compression circuit for subdividing and compressing continuous digital data into compressed data blocks and an expansion circuit for restoring compressed data to their original, expanded state.

EP-B1,0 129 981 describes a system for processing physiological signals, such as Holter ECG's, said system comprising devices for compressing data and restoring the original physiological signals for analysis.

US,A,4 223 678 describes a device for recording cardiac arrhythmias, ECG signals being continuously recorded and temporarily stored in an auxiliary memory. When a fibrillation is detected, the corresponding part of the ECG is downloaded to a main memory and therefrom transmitted by telemetry to another device for analysis outside the patient.

Common to the prior art methods according to these documents is thus the circumstance that the analysis is performed on the restored, decompressed information, with the disadvantages this entails, as discussed above.

The object of the present invention is to eliminate the disadvantages of the aforementioned technic.

This object is achieved with a method and a device of the kind mentioned in the introductory part with the characterizing features set forth in claims 1 and 7 respectively.

In the present invention the analysis of the information is thus performed on compressed data with retention of the possibility to restore the complete, original information. This will make the analysis much faster and more efficient. The analysis of the compressed information must obviously be performed according to other criteria than in the analysis of decompressed information. The analysis of compressed information conveys two important advantages, namely, firstly, the signal does not have to be decompressed, thereby eliminating the relatively long decompression times, and secondly, the signal analysis is considerably faster, since it is performed on a much smaller amount of information, i.e. one tenth of the total amount of information in the case of an Holter ECG. If a computer is used which is on a par with the current state of the art, the analysis operation will be performed much more rapidly, enabling the physician/operator to save considerable time and work more efficiently, alternately, a much simpler and cheaper computer (PC) could be used, thereby reducing the capital costs. Developing algorithms for the analysis of compressed ECG's is relatively simple for the man skilled in the art. During the test procedure, the algorithms can be easily verified by analysis also of the decompressed ECG in the previously known manner.

According to an advantageous further developemnt of the method according to the invention, compressed data can be decompressed when necessary for the physician's final analysis, the final analysis then being based on the results of the computer analysis of compressed data and on the original signals, restored by data decompression, alternatively interesting parts of the information determined by the computer analysis can be restored by decompression for further manual analysis.

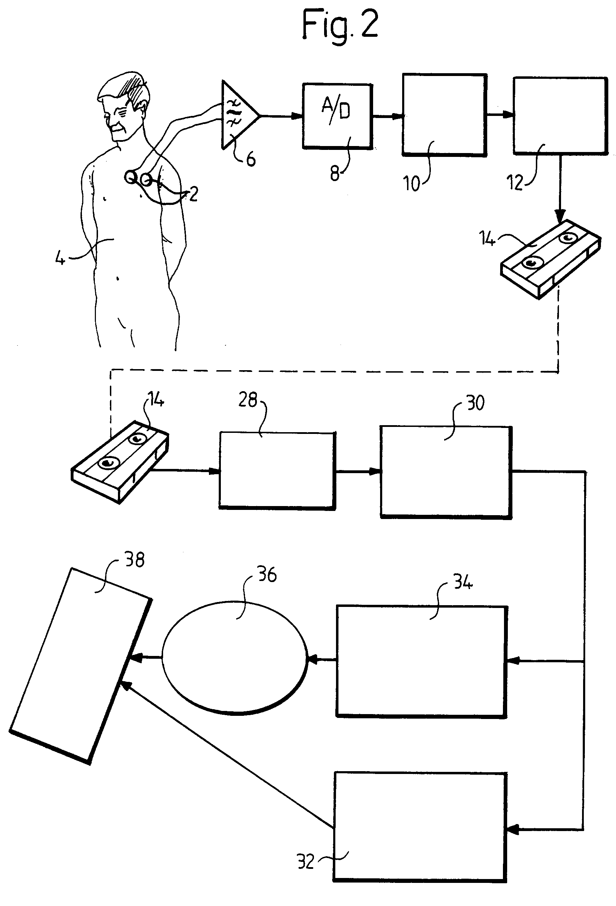

The invention will be now described below in greater detail, with reference to the attached FIGURE 2, with the aid of an embodiment of the device acoording to the invention, chosen as an example.

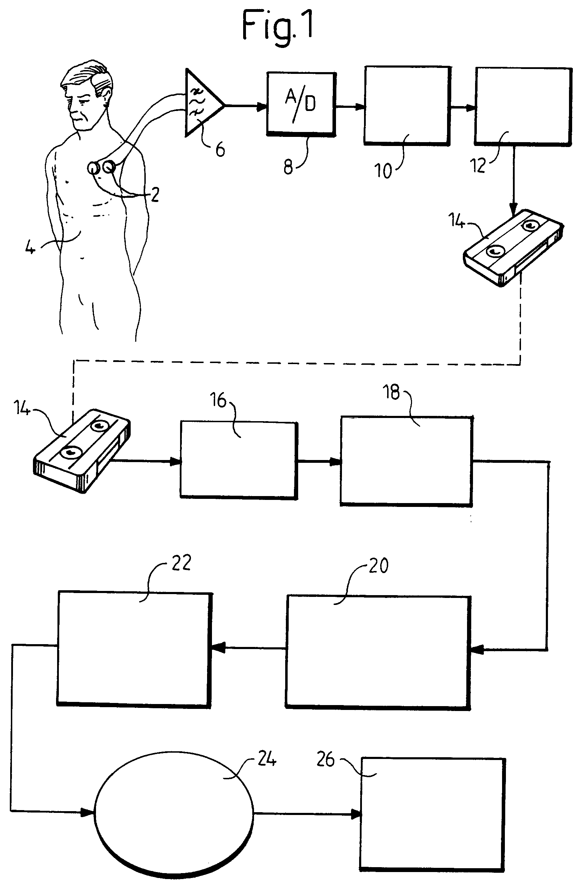

On the drawings FIG.1 shows a block diagram of a prior art device for recording and analyzing a Holter ECG, and FIG. 2 shows such a device according to the invention.

The upper part of FIG. 1 illustrates the equipment for recording a Holter ECG with electrodes 2 applied to the patient's 4 skin. This equipment is worn by the patient during the recording of the ECG. The signal picked up by the electrodes 2 is filtered and amplified in the filter and amplifier 6 and digitized in the A/D converter 8. The digitized information is compressed with the aid of a computer 10, the compressed information thereafter being stored, via the unit 12, on some appropriate medium, e.g. cassette tape 14.

Data compression is a well-known technic and need not be described more in detail. By data compression the amount of information is reduced to, typically, one tenth or less of the total amount of information. This reduces the cost of the equipment required, since the need for a.o. storage capacity is reduced to the corresponding degree, and memories are comparatively expensive.

Typically, about 20 Mb of storage space are needed for each ECG channel to store a complete 24-hour ECG.

The lower part of FIG. 1 shows a previously known equipment for replay and analysis of the recorded ECG.

Here, the ECG recorded on the cassette tape 14 is downloaded to a working memory 16, whereupon the information is decompressed with the aid of the computer 18 and supplied to an analysis station 20. In the analysis station 20 an automatic preanalysis is performed before the physician begins analyzing, editing and making a diagnosis from the data. The automatic preanalysis classifies different QRS morphologies and assigns all the QRS's to classes, the number of which can range from one or a few dozen up to 256 or more. The result of the preanalysis are issued via the unit 22, and the task of the physician then becomes to analyze the classes of QRS morphologies in order to interpret their significance, collect similar classes under main headings, such as "normal QRS", which originally could be found in 10-20 different classes with small morphological variations etc., at 24, and then the physician issues his final report, at 26.

The above-described method represents a considerable improvement of the originally completely manual procedure, in which the physician observed and analyzed every individual QRS, i.e. more than 100,000 QRS's per channel in a 24-hour recording, as noted above. The described, partially automatic process therefore considerably rationalizes the physician's work.

The disadvantage of the above-described prior technic resides in the fact that the same analysis as the physician would have performed is performed by a computer, i.e. classification of the appearance of the signal as originally recorded. For this to be done the compressed information must first be decompressed, which takes a long time.

In the embodiment of the present invention shown in FIG. 2 the recording equipment shown in the upper part of the figure is identical to the recording equipment according to the prior art, cf. FIG. 1, and the same reference designations are used as in FIG. 1.

In the replay and analysis equipment shown in the lower part of FIG. 2, the recorded information is downloaded from the cassette 14 to an working memory 28 and a computer 30 then performs an automatic preanalysis of the compressed information. This preanalysis is of course performed according to other criteria than those needed for the decompressed information. Developing the requisite algorithms for analyzing compressed ECG's present no difficulties for the man skilled in the art. In addition the algorithms can be easily verified by analyzing ECG's decompressed in the unit 32 for test purposes.

The preanalyzed information is issued via the report unit 34 and the physician then carries out editing, analysis and diagnosis, at 36, and issues his final report, at 38, in a manner analogous to the previously known technic according to FIG. 1.

By performing the analysis on the compressed signal primarily two important advantages are gained. Firstly, the signal does not have to be decompressed, thereby eliminating the long time required for decompression. Secondly, the signal analysis is much faster, since a greatly reduced amount of information needs to be processed, in the example mentioned above only one tenth of the total amount of information. With an equivalent computer, the analysis procedure will be much faster, and the physician/operator will save much time and be able to work more efficiently. Alternately, a simpler and cheaper computer can be used, thereby reducing the capital costs.

In certain instances, the physician's analysis and diagnosis may require restoration of a certain part of the ECG signal to its original form with the aid of the decompression unit 32, so the complete signal is available to the physician.

In the example above, the invention has been described as applied to Holter ECG data, but the technic can obviously be used for analysis of any analog signals processed as per the above.

## Claims

1. A method for analyzing large amounts of information, e.g. analysis of pictures, in which method information carrying signals are processed, and corresponding information data are compressed in such a way that the original signals can be recreated by data decompression, **characterized in** that the analysis is performed on the compressed data.

2. The method according to claim 1, **characterized in** that the analysis of compressed data is performed with the aid of a computer.

3. The method according to claim 2, **characterized in** that a manual final analysis is performed after the computer analysis.

4. The method according to claim 3, **characterized in** that compressed data are decompressed for the final analysis, and in that the final analysis is performed on the basis of the results of the computer analysis and of the original information restored by the decompression.

5. The method according to any one of the claims 2-4, **characterized in** that the parts of the information, identified as interesting by the computer analysis, are decompressed.

6. The method according to any one of the claims 1-5, the information carrying signals being analogue, **characterized in** that the signal processing prior to the data compression comprises amplification, filtration and A/D conversion.

7. A device for analyzing large amounts of information, e.g. analysis of pictures, comprising a unit for acquisition, compression and storage of the information, **characterized by** a unit for computer analysis of the compressed information.

8. The device according to claim 7, **characterized in** that the decompression unit is provided to decompress compressed data, after the computer analysis, to restore the original information or parts thereof.

9. The device according to claims 7 and 8, **characterized in** that the unit for computer analysis is arranged to determine interesting parts of the information, and in that the decompression unit is arranged to decompress these interesting parts of the information.

10. The device according to any one of the claims 7-9, the information consisting of physiological signals, such as ECG signals, recorded for a long period of time, i.e. Holter ECG, **characterized in** that the unit for computer analysis is arranged to classify QRS morphologies.

11. The device according to any one of the claims 7-10, **characterized in** that the unit for information acquisition, compression and storage comprises a filter, an amplifier and an A/D converter for filtering, amplifying and digitizing analog information signals.

12. The device according to claim 10 or 11, **characterized in** that the unit for information acquisition, compression and storage is part of portable recording equipment, and in that the unit for computer analysis is arranged in an analysis station separated from the recording equipment.
